(19) Europäisches Patentamt
European Patent Office
Office européen des brevets

(11) Veröffentlichungsnummer : **0 483 066 A2**

(12) **EUROPÄISCHE PATENTANMELDUNG**

(21) Anmeldenummer : **91810808.5**

(22) Anmeldetag : **18.10.91**

(51) Int. Cl.⁵ : **C08G 65/40,** C07C 211/46, C08L 79/08, C08L 63/00

(30) Priorität : **26.10.90 CH 3424/90**

(43) Veröffentlichungstag der Anmeldung : **29.04.92 Patentblatt 92/18**

(84) Benannte Vertragsstaaten : **DE ES FR GB IT NL SE**

(71) Anmelder : **CIBA-GEIGY AG**
**Klybeckstrasse 141**
**CH-4002 Basel (CH)**

(72) Erfinder : **Kramer, Andreas, Dr.**
**Bundtels 3**
**CH-3186 Düdingen (CH)**
Erfinder : **Vonlanthen, Christian**
**La Verdure**
**CH-1711 Ependes (CH)**

(54) **Lösliche Polyarylenether.**

(57) In organischen Lösungsmitteln lösliche, Aminogruppen aufweisende Polyarylenether mit einer inhärenten Viskosität von 0,02 bis 1,0, gemessen an einer 1 gew. -%igen Lösung in N-Methylpyrrolidon bei 25°C, die, bezogen auf die Gesamtmenge der im Polymeren vorhandenen Strukturelemente, 100 bis 5 Mol-% eines wiederkehrenden Strukturelementes der Formel I oder II

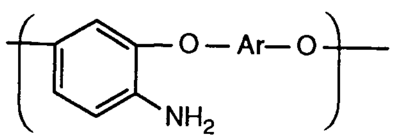

(I)

oder

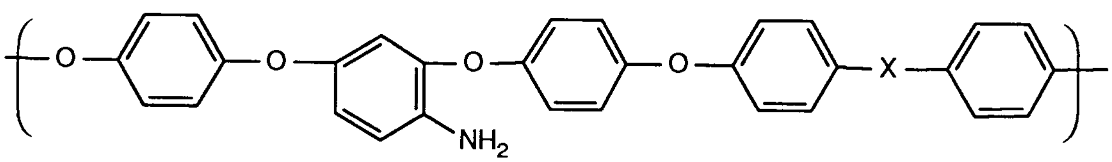

(II)

und 95 bis 0 Mol-% eines wiederkehrenden Strukturelementes der Formel III

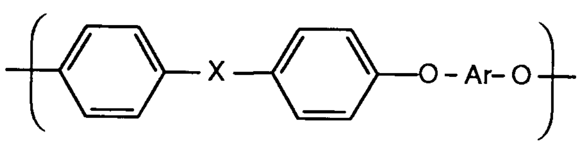

(III)

enthalten, worin
X für $-SO_2-$ oder -CO- steht und
Ar eine unsubstituierte oder durch ein oder mehrere $C_1$-$C_4$ -Alkyle, $C_1$-$C_4$ -Alkoxy oder Halogenatome substituierte Gruppe der Formeln IVa bis IVe

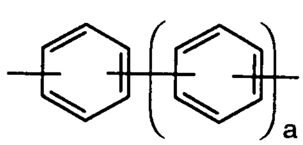

(IVa),

EP 0 483 066 A2

wobei a Null oder die Zahl 1 ist,

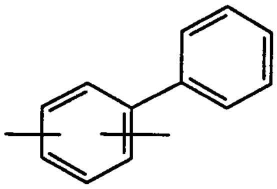 (IVb), 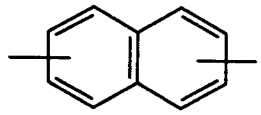 (IVc),

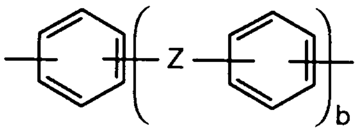 (IVd),

wobei b die Zahl 1 oder 2 ist, oder

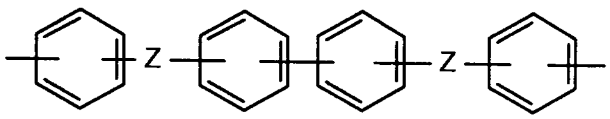 (IVe)

darstellt, worin Z für

-CO-, $-SO_2-$, -SO-, -S-, -O-, $-C(CH_3)_2$ , $-C(CF_3)_2$ , $-CH_2-$

oder

$-C-CH_3$
$C_6H_5$

steht, eignen sich vorzugsweise als Härtungs- oder Modifizerungsmittel für Maleinimid- oder Epoxidharze.

Die vorliegende Erfindung betrifft in organischen Lösungsmitteln lösliche und in der Polymerkette Aminogruppen aufweisende Polyarylenether, Verfahren zu deren Herstellung und deren Verwendung zum Modifizieren von duromeren Kunststoffen, insbesondere als Härtungs- oder Modifiziermittel für Maleinimid- und Epoxidharze.

Aminogruppen-terminierte Polymere, wie beispielsweise Aminogruppen-terminierte Poly-sulfone oder Polyarylenther, sind bekannt, beispielsweise aus dem US-Patent 3,895,064 und der EP-A-0 311 349. Solche Aminogruppen-terminierte Polymere eignen sich für die Flexibilisierung von Epoxidharzen, wobei die daraus hergestellten Formstoffe gegenüber den Formstoffen aus nichtflexibilisierten Epoxidharzen meistens einen mehr oder weniger grossen Abfall beim Tg-Wert aufweisen, da bei der Härtung eine Phasentrennung eintritt, wie beispielsweise von R.B. Bauer et al. am 35th International SAMPE Symposium, April 2-5, 1990, Seiten 395 bis 403, berichtet wird.

Der im US-Patent 4,108,837, Beispiel 23, aus Bisphenol A und 1,3-Dichlor-4-nitrobenzol hergestellte Polyarylenether wird zur Herstellung von Filmen aus der Schmelze verwendet.

Aus der Publikation " Die Angewandte Makromolekulare Chemie" 119 (1983), Seiten 105-123, geht hervor, dass das aus Bisphenol A und 1,3-Dichlor-4-nitrobenzol (2,4-Dichlornitrobenzol) erhaltene Polymer mit Raney-Nickel und Hydrazin nur unvollständig chemisch hydriert wird, und dass das nach der Reduktion isolierte Reaktions-produkt in organischen Lösungsmitteln, wie N-Methyl-2-pyrrolidon, Dimethylsulfoxid, Tetrahydrofuran, Petrolether und Dimethylacetamid, unlöslich ist. Damit nach dem Wegfall der Hydrierbedingungen keine vorzeitige Vernetzung eintritt, werden die Aminogruppen mit einer Schutzgruppe versehen.

Es wurde nun gefunden, dass man durch katalytische Hydrierung eines Nitrogruppen aufweisenden Polykondensates aus 1,3-Dichlor-4-nitrobenzol und Bisphenol A mit Wasserstoff unter Normaldruck in Gegenwart von Pd-Kohle ein Aminogruppen aufweisendes Polymer erhält, das in organischen Lösungsmitteln, wie Methylenchlorid, Tetrahydrofuran, Dimethylformamid oder N-Methylpyrrolidon, sehr gut löslich ist und mit diesen Lösungsmitteln sehr gute stabile Lösungen bildet.

Gegenstand der vorliegenden Erfindung sind somit in organischen Lösungsmitteln lösliche, Aminogruppen aufweisende Polyarylenether mit einer inhärenten Viskosität ($\eta_{inh}$) von 0,02 bis 1,0, gemessen an einer 1 gew.-%igen Lösung in N-Methylpyrrolidon bei 25°C, die, bezogen auf die Gesamtmenge der im Polymeren vorhandenen Strukturelemente, 100 bis 5 Mol-% eines wiederkehrenden Strukturelementes der Formel I oder II

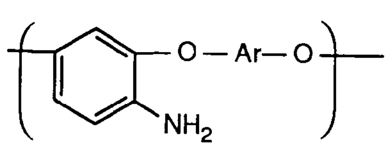

(I)

oder

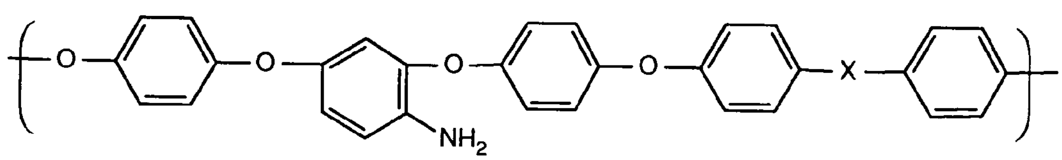

(II)

und 95 bis 0 Mol-% eines wiederkehrenden Strukturelementes der Formel III

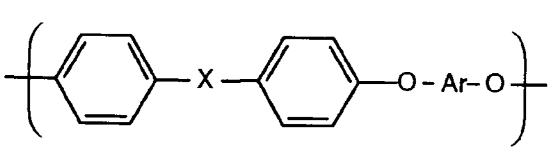

(III)

enthalten, worin
X für $-SO_2-$ oder -CO- steht und
Ar eine unsubstituierte oder durch ein oder mehrere $C_1$-$C_4$ -Alkyle, $C_1$-$C_4$ -Alkoxy oder Halogenatome substituierte Gruppe der Formeln IVa bis IVe

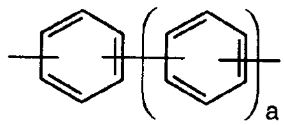 (IVa),

wobei a Null oder die Zahl 1 ist,

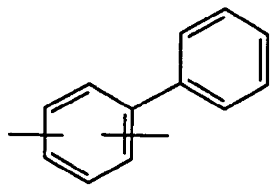 (IVb),

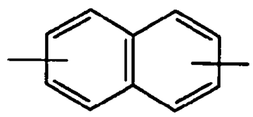 (IVc),

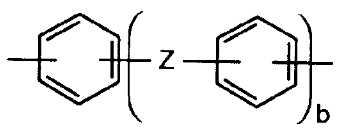 (IVd),

wobei b die Zahl 1 oder 2 ist, oder

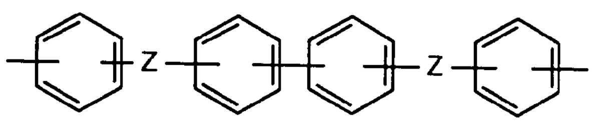 (IVe)

darstellt, worin Z für

$$-CO-,\ -SO_2-,\ -SO-,\ -S-,\ -O-,\ -\overset{|}{C}(CH_3)_2\ ,\ \ -\overset{|}{C}(CF_3)_2\ ,\ -CH_2-$$

oder

$$\begin{array}{l} \ \ | \\ -C-CH_3 \\ \ \ | \\ \ C_6H_5 \end{array}\ \text{steht.}$$

Vorzugsweise enthalten die erfindungsgemässen Polyarylenether 100 bis 5 Mol- % eines wiederkehrenden Strukturelementes der Formel I und 95 bis 0 Mol-% eines wiederkehrenden Strukturelementes der Formel III. Insbesondere sind in den erfindungsgemässen Polyarylenethern 100 bis 10 Mol-% eines wiederkehrenden Strukturelementes der Formel I und 90 bis 0 Mol-% eines wiederkehrenden Strukturelmentes der Formel III enthalten.

Die erfindungsgemässen Polyarylenether werden physikalisch durch ihre inhärente Viskosität ($\eta_{inh}$) charakterisiert, welche bekanntlich ein Mass für das Molekulargewicht eines Polymeren darstellt. Die $\eta_{inh}$ der erfindungsgemässen Polyarylenether erstreckt sich über einen Bereich von 0,02 bis 1,0. Dies entspricht einem numerischen Molekulargewicht (Mn) von etwa 500 bis 20 000 (Zahlenmittel). Bevorzugt sind Polyarylenether mit einem Mn von 500 bis 15 000, was einer $\eta_{inh}$ von etwa 0,02 bis 0,7 entspricht.

In den erfindungsgemässen Polyarylenethem bedeutet Ar in den Formeln I und III vorzugsweise eine unsubstituierte Gruppe der Formeln IVa bis IVe.

Der Rest Ar in den Strukturelementen der Formeln I und III steht vorzugsweise für einen Rest der Formeln

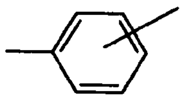 , 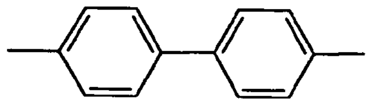 , 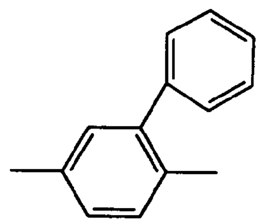 ,

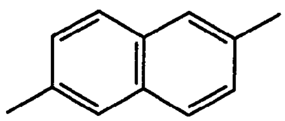 , 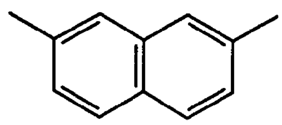 , 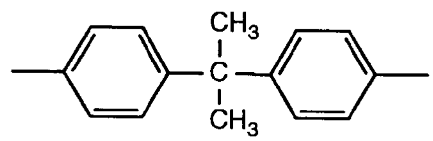

oder

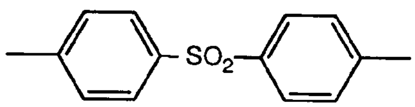 .

Insbesondere steht der Rest Ar in den Strukturelrmenten der Formeln I und III für einen Rest des Formeln

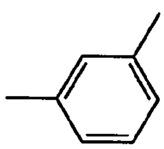 , 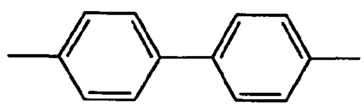 , 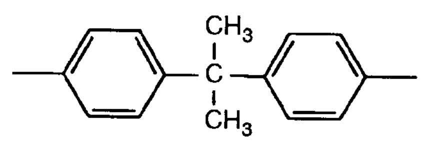 ,

oder

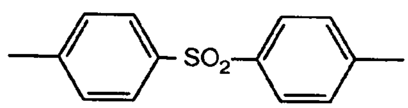 .

Die erfindungsgemässen Polyarylenether können hergestellt werden, indem man beispielsweise 1,3-Dichlor-4-nitrobenzol oder ein Gemisch aus 1,3-Dichlor-4-nitrobenzol und einer darin bis zu 95 Mol-%, vorzugsweise 90 Mol-%, enthaltenen Dihalogenverbindung der Formel V

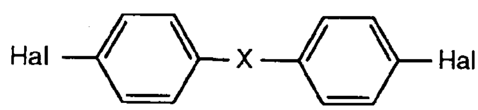 (V),

worin Hal für ein Halogenatom, vorzugsweise Chlor oder Fluor, steht und X die oben angegebene Bedeutung hat, mit einem Diphenol der Formel VI

HO-Ar-OH (VI)

worin Ar die oben angegebene Bedeutung hat, oder indem man 2,4-Bis-(4-hydroxy-phenoxy)-anilin oder ein Gemisch aus 2,4-Bis-(4-hydroxyphenoxy)-anilin und einem darin bis zu 95 Mol-%, vorzugsweise 90 Mol-%, enthaltenen Diphenol der Formel VI mit einer Halogenverbindung der Formel V in Gegenwart von Alkali und in einem aprotischen Lösungsmittel polykondensiert, bis der erhaltene Polyarylenether eine $\eta_{inh}$ von 0,02 bis 1,0 aufweist, und anschliessend den Nitrogruppen enthaltenden Polyarylenether durch vollständige katalytische Reduktion der Nitrogruppen in einen erfindungsgemässen, Aminogruppen aufweisenden Polyarylenether umwandelt.

Vorzugsweise wird bei diesem Herstellungsverfahren das Diphenol der Formel VI oder das Gemisch aus 2,4-Bis-(4-hydroxyphenoxy)-anilin und einem Diphenol der Formel VI im äquivalenten Ueberschuss eingesetzt.

Als Alkali verwendet man in diesem Verfahren in der Regel Alkali- und Erdalkalicarbonate, wie Natrium-, Kalium- oder Calciumcarbonat; doch können auch andere alkalische Reagentien, wie Natriumhydroxid, Kaliumhydroxid oder Calciumhydroxid, Verwendung finden.

Polare, aprotische Lösungsmittel, die beim Verfahren zur Herstellung der erfindungsgemässen Polyarylenetherharze eingesetzt werden können, sind beispielsweise Diethylacetamid, Tetramethylharnstoff, N-Methylcaprolactam und bevorzugt Dimethylacetamid oder N-Methylpyrrolidon.

Die Reaktion wird zweckmässig bei erhöhter Temperatur durchgeführt, vorzugsweise bis zur Rückflusstemperatur des Lösungsmittels, also etwa bis 250°C.

Es empfiehlt sich die Mitverwendung eines Schleppmittels, wie z.B. Chlorbenzol, Xylol oder Toluol, um das bei der Umsetzung gebildete Wasser azeotrop aus dem Reaktionsgemisch entfernen zu können.

Das 1,3-Dichlor-4-nitrobenzol ist bekannt und im Handel erhältlich, beispielswese von der Fluka Chemie AG, Buchs, CH.

Die Dihalogenverbindungen der Formel V sind auch bekannt, beispielsweise aus der DE-OS 30 14 230 und der EP-A-0 001879. Geeignete Dihalogenverbindungen der Formel V sind beispielsweise 4,4′-Dichlordiphenylsulfon, 4,4′-Difluordiphenylsulfon, 4,4′-Dichlorbenzophenon und 4,4′-Difluorbenzophenon.

Die Diphenolverbindungen der Formel VI stellen ebenfalls bekannte Verbindungen dar und sind grösstenteils im Handel erhältlich. Beispiele für geeignete zweiwertige Phenole der Formel VI sind Hydrochinon, Resorcin, 4,4′-Dihydroxybiphenyl, 2,5-Dihydroxybiphenyl, 4,4′-Dihydroxydiphenylether, 4,4′-Dihydroxydiphenylsulfon, 4,4′-Dihydroxy-3,3′,5,5′-tetramethyldiphenylsulfon, 4,4′-Dihydroxybenzophenon, 4,4′-Dihydroxydiphenylmethan, 4,4′-Dihydroxydiphenylthioether, 2,2-Di-(4-hydroxyphenyl)-propan oder Dihydroxynaphthalin. Ferner sind 2,6-Bis-(4-hydroxybenzoyl)-naphthalin und 2,7-Bis-(4-hydroxybenzoyl)-naphthalin aus der DE-OS 38 04159 bekannt, und chlor- oder methylsubstituiertes 2,6-Bis-(4-hydroxybenzoyl)-naphthalin aus den US-Patenten 4 447 592 und 4 275 226.

Das ebenfalls zur Herstellung der erfindungsgemässen Polyarylenether verwendete 2,4-Bis-(4-hydroxyphenoxy)-anilin ist in der Literatur noch nicht beschrieben worden und kann beispielsweise hergestellt werden, indem man 1,3-Dichlor-4-nitrobenzol mit überschüssigem Hydrochinonmonobenzylether in Gegenwart von Alkali und in einem aprotischen Lösungsmittel, wie beispielsweise in N-Methylpyrrolidon, umsetzt und die erhaltene Nitroverbindung in bekannter Weise zu 2,4-Bis-(4-hydroxyphenox)-anilin hydriert. Das für die erfindungsgemässen Polyarylenether hergestellte 2,4-Bis-(4-hydroxy-phenoxy)-anilin stellt somit ebenfalls einen Erfindungsgegenstand dar.

Wie eingangs erwähnt, sind die erfindungsgemässen Polyarylenether in gebräuchlichen organischen Lösungsmitteln, vorzugsweise in halogenierten, d.h., chlorierten oder fluorierten, Kohlenwasserstoffen, insbesondere in chlorierten Kohlenwasserstoffen, wie beispielsweise Methylenchlorid, Tri- oder Tetrachlormethan oder Chlorbenzol löslich. Die erfindungsgemässen Polyarylenether sind auch in polaren aprotischen Lösungsmitteln, wie N-Methylpyrrolidon, N,N-Dimethylformamid und Dimethylsulfoxid, oder in cyclischen Ethern, wie Tetrahydrofuran oder Dioxan, sowie auch in Cyclohexanon löslich. Aufgrund ihrer Löslichkeit können die erfindungsgemässen Polyarylenether vorteilhaft aus einer Lösung heraus zu Filmen verarbeitet oder in andere Matrixsysteme eingebracht werden. Die Lösungen der erfindungsgemässen Polyarylenether sind über mehrere Wochen stabil, das heisst, es erfolgt weder eine Trübung, ein Niederschlag oder die Ausfällung des Polymeren.

Vor der Verarbeitung der beispielsweise als Schmelze oder insbesondere als Lösung vorliegenden Polyarylenether können übliche Zusatzstoffe, wie beispielsweise Füllstoffe, Pigmente, Stabilisatoren oder Verstärkungsmittel, wie Kohlenstoff-, Bor-, Metall- oder Glasfasern, zugegeben werden.

Die erfindungsgemässen Polyarylenether können zum Härten und Modifizieren von Maleinimid- oder Epoxidharzen, insbesondere von Epoxidharzen, eingesetzt werden. Diese Verwendung stellt einen weiteren Erfindungsgegenstand dar.

Die Verwendung von Polyaminen zum Härten oder Modifizieren von Maleinimidharzen, wie Mono- oder Polymaleinimiden, ist bekannt, beispielsweise aus dem FR-Patent 1 555 564 oder der DE-OS 2 350 471. Als

Maleinimidharze, die mit den erfindungsgemässen Polyarylenethem gehärtet oder modifiziert werden können, eignen sich beispielsweise Verbindungen der Formel VII

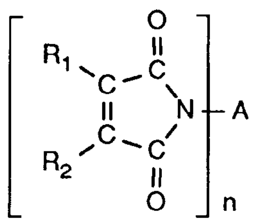

(VII),

worin $R_1$ und $R_2$ unabhängig voneinander je ein Wasserstoffatom oder eine lineare oder verzweigte Alkylgruppe mit 1 bis 4 C-Atomen und A einen n-wertigen aliphatischen Rest mit 2 bis 30 C-Atomen, einen cycloaliphatischen, einen aromatischen, einen heterocyclischen oder einen araliphatischen Rest bedeuten und n für 1, 2, 3 oder 4 steht, oder A auch, wenn n für 2 steht, die direkte Bindung bedeutet.

Insbesondere eignen sich die folgenden Maleinimide:
N,N′-Ethylen-bis-maleinimid, N,N′-Hexamethylen-bis-maleinimid, N,N′-m-Phenylen-bis-maleinimid, N,N′-p-Phenylen-bis-maleinimid, N,N′-4,4′-Diphenylmethan-bis-maleinimid, N,N′-4,4′-3,3′-Dichlor-diphenylmethan-bis-maleinimid, N,N′-Diphenylethan-bis-maleinimid, N,N′-4,4′-Diphenylsulfon-bis-maleinimid, N,N′-m-Xylylen-bis-maleinimid, N,N′-p-Xylylen-bis-maleinimid, N,N′-4,4′-2,2-Diphenylpropan-bis-maleinimid, das N,N′-Bis-maleinimid des 4,4′-Diamino-triphenylphosphats, das N,N′-Bis-maleinimid des 4,4′-Diamino-triphenylphosphits, das N,N′-Bis-maleinimid des 4,4′-Diamino-triphenylthiophosphats, das N,N′,N″-Trismaleinimid des Tris-(4-aminophenyl)-phosphats, das N,N′,N″-Trismaleinimid des Tris-(4-aminophenyl)-phosphits und das N,N′,N″-Trismaleinimid des Tris-(4-aminophenyl)-thiophosphats.

Polyamine stellen bekannte Härtungs- und Modifizierungsmittel für Epoxidharze dar. Die erfindungsgemässen Polyarylenether, welche mit Epoxidharzen gut verträglich sind und ein homogenes einphasiges System bilden, werden vorzugsweise zum Härten oder Modifizieren von Epoxidharzen eingesetzt, wobei eine Flexibilisierung des gehärteten Epoxidharzes mit relativ nur geringem Abfall des Tg-Wertes erreicht wird. Gegenstand der Erfindung ist somit auch ein härtbares Gemisch, enthaltend (a) ein Epoxidharz mit mehr als einer Epoxidgruppe im Molekül und (b) einen erfindungsgemässen Polyarylenether in für die Aushärtung des Epoxidharzes ausreichender Menge.

Als Epoxidharz (a) eignen sich alle Typen von Epoxidharzen, wie beispielsweise solche, die direkt an Sauerstoff-, Stickstoff- oder Schwefelatome gebundene Gruppen der Formel VIII

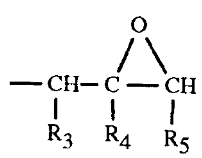

(VIII),

worin entweder $R_3$ und $R_5$ je ein Wasserstoffatom darstellen, in welchem Fall $R_4$ dann ein Wasserstoffatom oder eine Methylgruppe bedeutet, oder $R_3$ und $R_5$ zusammen $-CH_2CH_2-$oder $-CH_2CH_2CH_2-$ darstellen, in welchem Fall $R_4$ dann ein Wasserstoffatom bedeutet, enthalten.

Als Beispiele solcher Harze seien Polyglycidyl- und Poly-(β-methylglycidyl)-ester genannt, die man durch Umsetzung einer zwei oder mehrere Carbonsäuregruppen pro Molekül enthaltenden Verbindung mit Epichlorhydridn, Glycerindichlorhydrin oder β-Methylepichlorhydrin in Gegenwart von Alkali erhalten kann. Solche Polyglycidylester können sich von aliphatischen Polycarbonsäuren, z.B. Oxalsäure, Bernsteinsäure, Glutarsäure, Adipinsäure, Pimelinsäure, Korksäure, Azelainsäure, Sebacinsäure oder dimerisierter oder trimerisierter Linolsäure, von cycloaliphatischen Polycarbonsäuren, wie Tetrahydrophthalsäure, 4-Methyltetrahydrophthalsäure, Hexahydrophthalsäure und 4-Methylhexahydrophthalsäure, und von aromatischen Polycarbonsäuren, wie Phthalsäure, Isophthalsäure und Terephthalsäure, ableiten.

Weitere Beispiele sind Polyglycidyl- und Poly- (β-methylglycidyl)-ether, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung, erhältlich sind. Diese Ether lassen sich mit Poly-(epichlorhydrin) aus acyclischen Alkoholen wie Ethylenglykol, Diethylenglykol und höheren Poly-(oxyethylen)-glykolen, Propan-1,2-diol und Poly-(oxypropylen)-glykolen, Propan-1,3-diol, Butan- 1,4-diol, Poly-(oxytetramethylen)-glykolen, Pentan-1,5-diol, Hexan- 1,6-diol, Hexan-2,4,6-triol, Glycerin, 1,1,1 -Trimethylpropan, Pentaerythrit und Sorbit, aus cycloaliphatischen Alkoholen, wie Resorcit, Chinit, Bis-(4-hydroxycyclohexyl)-methan, 2,2-Bis-(4-hydroxycyclohexyl)-propan und 1, 1-Bis-(hydroxymethyl)-cyclohexen-3, und aus Alkoholen mit aromatischen Kernen, wie N,N-Bis-(2-hydroxyethyl)-anilin und p,p′-Bis-(2-hydroxyethylamino)-diphenylmethan, herstellen. Man kann sie ferner aus einkemigen Phenolen, wie Resorcin und Hydrochinon, sowie mehrkenigen Phenolen, wie Bis-(4-hydroxyphenyl)-methan, 4,4-Dihydroxydiphenyl, Bis-(4-hydroxyphenyl)-sulfon, 1, 1,2,2,-Tetrakis-(4-hydroxyphenyl)-ethan, 2,2-Bis-(4-hydroxyphenyl)-propan (sonst als Bisphenol A bekannt) und 2,2-Bis-(3,5-dibrom-4-hydroxyphenyl)-propan sowie aus Aldehyden, wie Formaldehyd, Acetaldehyd, Chloral und Furfural, mit Phenolen, wie Phenol selbst und durch Chloratome oder Alkylgruppen mit jeweils bis zu neun Kohlenstoffatomen ringsubstituiertem Phenol, wie 4-Chlorphenol, 2-Methylphenol und 4-tert.-Butylphenol, gebildeten Novolaken herstellen.

Poly-(N-glycidyl)-verbindungen umfassen beispielsweise Triglycidylisocyanurat, N,N,N′,N′-Tetraglycidyl-4,4′-diaminodiphenylmethan, N,N,N′,N′-Tetraglycidyl-3,3′-dialkyl-4,4′-diaminodiphenylmethane sowie N,N′-Diglycidylderivate von cyclischen Alkylenhamstoffen, wie Ethylenharnstoff und 1,3-Propylenharnstoff, und Hydantoinen, wie 5,5-Dimethylhydantoin.

Poly-(S-glycidyl)-verbindungen sind zum Beispiel die Di-S-glycidylderivate von Dithiolen, wie Ethan- 1,2-dithiol und Bis-(4-mercaptomethylphenyl)-ether.

Beispiele für Epoxidharze mit Gruppen der Formel VIII, worin $R_3$ und $R_5$ zusammen eine $-CH_2-CH_2-$Gruppe bedeuten, sind Bis-(2,3-epoxycyclopentyl)-ether, 2,3-Epoxycyclopentyl-glycidylether, 1,2-Bis-(2,3-epoxycyclopentyloxy)-ethan und 3,4-Epoxycyclohexylmethyl-3′,4′-epoxycyclohexancarboxylat.

In Betracht kommen auch Epoxidharze, in welchen die 1,2-Epoxidgruppen an Hetero-atome verschiedener Art gebunden sind, z.B. das N,N,O-Triglycidylderivat des 4-Aminophenols, der Glycidylether/Glycidylester der Salicylsäure oder p-Hydroxybenzoesäure, N-Glycidyl-N′-(2-glycidyloxypropyl)-5,5-dimethylhydantoin und 2-Glycidyloxy- 1,3-bis-(5,5-dimethyl-1-glycidylhydantoinyl-3)-propan.

Speziell bevorzugt werden aromatische Epoxidharze, wie Novolak-Epoxidharze, Diglycidylether von Bisphenolen oder Tetraglycidylverbindungen von aromatischen Diaminen.

Die erfindungsgemässen Polyarylenether (b) liegen im härtbaren Epoxidharzgemisch dann in für die Aushärtung ausreichender Menge vor, wenn im Gemisch auf 1 Epoxidäquivalent etwa 0,8 bis 1,2 Aminwasserstofäquivalente vorhanden sind.

Wie bereits erwähnt, ist es auch möglich, die erfindungsgemässen Polyarylenether zum Modifizieren von Epoxidharzen einzusetzen. In einem solchen Fall liegt im härtbaren Epoxidharzgemisch aus (a) und (b) ein weiteres von (b) verschiedenes Epoxidharzhärtungsmittel (c) vor, wobei einerseits (b) und (c) zusammen in für die Aushärtung des Epoxidharzes ausreichender Menge vorliegen oder andererseits nur (c) in für die Aushärtung des Epoxidharzes ausreichender Menge vorliegt und der Polyarylenether (b) in Mengen bis zu 50 Gew.-%, bezogen auf die Menge (a) und (c), vorliegt. Als von (b) verschiedene Epoxidharzhärtungsmittel (c) können beispielsweise andere Amine, einschliesslich der aliphatischen, cycloaliphatischen, aromatischen und heterocyclischen Amine, verwendet werden, wie m- und p-Phenylendiamin, Bis-(4-aminophenyl)-methan, Anilin-Formaldehyd-Harz, Bis-(4-aminophenyl)-sulfon, Ethylendiamin, Propan- 1,2-diamin, Propan- 1,3-diamin, N,N-Diethylethylendiamin, Hexamethylendiamin, Diethylentriamin, Triethylentetramin, Tetraethylenpentamin, N-(2-Hydroxyethyl)-, N-(2-Hydroxypropyl)-, und N-(2-Cyanoethyl)-diethylentriamin, 2,2,4-Trimethylhexan- 1,6-diamin, 2,3,3-Trimethylhexan- 1,6-diamin, m-Xylylendiamin, N,N-Dimethyl- und N,N-Diethylpropan- 1,3-diamin, Aethanolamin, Bis-(4-aminocyclohexyl)-methan, 2,2-Bis-(4-aminocyclohexyl)-propan, 2,2-Bis-(4-amino-3-methylcyclohexyl)-propan, 3-Aminomethyl-3,5,5-trimethylcyclohexylamin (Isophorondiamin), und N-(2-Aminoethyl)-piperazin; sowie Dicyandiamid; Polyaminoamide, wie beispielsweise solche aus aliphatischen Polyaminen und dimerisierten oder trimerisierten Fettsäuren, aus Aminen mit einem stöchiometrischen Unterschuss an Polyepoxiden hergestellte Amin-Addukte; Isocyanate und Isothiocyanate; Polyphenole, wie Resorcin, Hydrochinon, 2,2-Bis-(4-hydroxyphenyl)-propan, Phenol-Aldehyd-Harze und ölmodifizierte Phenol-Aldehyd-Harze, Phosphorsäure, Polythiol, wie die im Handel unter der Bezeichnung "Thiokole " erhältlichen Polythiole, Polycarbonsäuren und ihre Anhydride, wie zum Beispiel Phthalsäureanhydrid, Tetrahydrophthalsäureanhydrid, Methylendomethylentetrahydrophthalsäureanhydrid, Nonenylbemsteinsäureanhydrid, Dodecenylbernsteinsäureanhydrid, Hexahydrophthalsäureanhydrid, Hexachloroendomethylentetrahydrophthalsäureanhydrid und Endomethylentetrahydrophthalsäureanhydrid und ihre Mischungen, Maleinsäureanhydrid, Bernsteinsäureanhydrid, Pyromellitsäureanhydrid, Benzophenon-3,3′,4,4′-tetracarbonsäuredianhydrid, Polysebazinsäureanhydrid, Polyazelainsäureanhydrid, die Säure der zuvorgenannten Anhyride sowie auch Isophthalsäure, Terephthalsäure, Zitronensäure und Mellitsäure. Besonders bevorzugte Polycarbonsäuren oder Anhydride sind solche, die unterhalb von 60°C flüssig sind. Es können auch katalytisch wirkende Härtungsmittel verwendet werden, wie beispielsweise tertiäre Amine (zum Beispiel 2,4,6-Tris-(dimethylaminoethyl)-phenol und andere Mannichbasen, N-Benzyldimethylamin und Triethanolamin); Akalimetallakoxide von Alkoholen (zum Beispiel Na-Alkoholat von 2,4-Dihydroxy-3-hydroxymethylpentan), Zinnsalze von Alkansäuren (zum Beispiel Zinnoctanoat), Friedel-Crafts-Katalysatoren, wie Bortrifluorid und seine Komplexe und Chelate, die durch Umsetzung von Bortrifluorid mit zum Beispiel 1,3-Diketonen erhalten werden.

Mit den Härtungsmitteln können auch die geeigneten Härtungsbeschleuniger eingesetzt werden. Bei Verwendung von Poly-(aminoamiden), Dicyandiamiden, Polythiolen oder Polycarbonsäureanhydriden können tertiäre Amine oder deren Salze, quaternäre Ammoniumverbindungen oder Alkalimetallakoxide als Beschleuniger dienen. Beispiele von bestimmten Beschleunigern sind N-Benzyldimethylamin, 2,4,6-Tris-(dimethylaminomethyl)-phenol, Imidazole und Triamylammoniumphenoxid.

Wenn das Härtungsmittel eine Polycarbonsäure oder deren Anhydrid ist, verwendet man gewöhnlich 0,6 bis 1,1 Aequivalente Carboxylgruppe bzw. Anhydridgruppe per 1 Aequivalent Epoxidgruppe. Bei der Verwendung von Polyphenolen als Härtungsmittel, setzt man zweckmässig 0,75 bis 1,25 Aequivalente phenolische Hydroxylgruppen per 1 Epoxidäquivalent ein. Katalytisch wirkende Härtungsmittel werden allgemein in Mengen von 1 bis 40 Gewichtsteilen pro 100 Gewichtsteile Epoxidharz eingesetzt.

Je nach Natur des verwendeten Härtungsmittels kann die Härtung bei Raumtemperatur oder bei höheren Temperaturen vorgenommen werden.

Verwendet man als Härtungsmittel ein Gemisch von verschiedenen Härtungsmitteln, so weiss der auf dem Epoxidharzgebiet tätige Fachmann zu ermitteln, welche Mengen an Härtungsmitteln und welche Härtungstemperaturen erforderlich sind, um eine vollständige Aushärtung des Epoxidharzes zu erreichen.

Die erfindunbsgemässen härtbaren Gemische können auch weitere, bekannte und üblicherweise in der Technik polymerisierbarer Materialien eingesetzte Zusatzstoffe enthalten. Beispiele für solche Zusatzstoffe sind Pigmente, Farbstoffe, Füllstoffe und Verstärkungsmittel, Flammhemmstoffe, Antistatika, Haftvermittler, Verlaufmittel, Antioxidantien und Lichtschutzmittel. Als Füllstoffe eignen sich mineralische und faserförmige Füllstoffe, wie Quarzmehl, Quarzgut, Aluminiumoxid, Glaspulver, Glimmer, Kaolin, Dolomit, Graphit, Russ, sowie Kohlefasern und Textilfasern. Bevorzugte Füllstoffe sind Quarzmehl, Quarzgut, Aluminiumoxid oder Dolomit.

Die erfindungsgemässen härtbaren Gemische lassen sich ganz allgemein zur Herstellung von gehärteten Produkten einsetzen und können in der dem jeweils speziellen Anwendungsgebiet angepassten Formulierung, beispielsweise als Beschichtungsmasssen, Lacke, Pressmassen, Tauchharze, Giessharze, Imprägnierharze, Laminierharze, 1- oder 2-Komponenten-Klebstoffe oder Matrixharze, eingesetzt werden.

Zur Aushärtung der erfindungsgemässen Gemische wendet man im allgemeinen Temperaturen im Bereich von 60 bis 250°C, vorzugsweise von 80 bis 220°C, insbesondere von 100 bis 200°C, an.

Man kann an den erfindungsgemässen Gemischen auch erst eine Vorhärtung bei tieferen Temperaturen bis zum Gelieren der härtbaren Zusammensetzung durchführen, an die sich dann eine Aushärtung bei höheren Temperaturen anschliesst.

Die aus den erfindungsgemässen Gemischen durch thermische Härtung erhaltenen Produkte zeichnen sich vor allem durch eine hohe Wärmeformbeständigkeit aus.

Herstellung von 2,4-Bis-(4-hydroxyphenoxy)-anilin

a) In einem 2,5 Liter Sulfierkolben werden 348,3 g (1,73 Mol) Hydrochinon-monobenzylether und 167 g (0,87 Mol) 1,3-Dichlor-4-nitrobenzol in Gegenwart von 293 g (1,90 Mol) Kaliumcarbonat (wasserfrei) in 870 ml N-Methyl-2-pyrrolidon (NMP) und 700 ml Xylol bei 147°C zur Reaktion gebracht und das entstehende Reaktionswasser wird während 8 Stunden unter Stickstoff azeotrop ausgekreist. Anschliessend wird das Xylol aus dem Reaktionsgefäss abdestilliert und die verbleibende Suspension nach Abkühlen auf Raumtemperatur auf 10 Liter Wasser gegossen. Der braune Niederschlag wird abfiltriert, dann in 4 Liter Methanol aufgeschlämmt, filtriert und unter Vakuum bei 80°C getrocknet. Das Rohprodukt wird aus 2,5 Liter Isopropanol umkristallisiert. Man erhält 280 g (62 % der Theorie) eines hellbraunen, kristallinen Pulvers mit einem Schmelzpunkt von 113-115°C.

| Elementaranalyse: | berechnet | gefunden |
|---|---|---|
| | 73,98 % C | 73,79 % C |
| | 4,85 % H | 4,88 % H |
| | 2,70 % H | 2,54 % N. |

b) In eine Suspension von 25 g 5%iger Pd-Kohle in 2,5 Liter Dimethylformamid werden 256 g der unter a) beschriebenen Nitroverbindung während 14 Stunden bei 37°C in Gegenwart von Wasserstoff (Normaldruck) hydriert. Anschliessend wird die Reaktionslösung filtriert und das Filtrat am Rotationsverdampfer bei 100°C eingeengt. Der Rückstand wird 2 mal in je 500 ml Methylenchlorid aufgekocht und anschliessend im Vakuum bei 70°C getrocknet. Man erhält in quantiativer Ausbeute ein violettes Pulver mit einem Phenolgehalt von 6,28 Aequivalenten/kg (Theorie: 6,47 Aequivalenten/kg) und einem Amingehalt von 3,07 Aequivalenten/kg (Theorie: 3,23 Aequivalenten/kg).

| Elementaranalyse: | berechnet | gefunden |
|---|---|---|
| | 69,89 % C | 68,53 % C |
| | 4,89 % H | 4,96 % H |
| | 4,53 % H | 4,34 % N |

Beispiel 1:

a) In einem 750 ml Sulfierkolben, ausgerüstet mit Rührer, Thermometer, Wasserabscheider, Kühler und Gaseinleitungsrohr, werden 96 g (0,5 Mol) 1,3-Dichlor-4-nitrobenzol, 114 g (0,5 Mol) Bisphenol A, 152 g (1,1 Mol) wasserfreies, pulverisiertes Kaliumcarbonat, 300 ml NMP und 180 ml Xylol zum Rückfluss erhitzt und das entstehende Reaktionswasser wird während 6-20 Stunden unter Stickstoff azeotrop ausgekreist. Anschliessend wird das Xylol aus dem Reaktionsgefäss abdestilliert und die verbleibende Suspension nach Abkühlen auf Raumtemperatur auf 3 Liter Wasser gegossen. Der Niederschlag wird abfiltriert, ein zweites Mal mit 5 Liter Wasser gemischt, filtriert, mit Wasser gut nachgewaschen und im Vakuum bei 100°C getrocknet. Man erhält 155 g eines beigen Pulvers, das in Methylenchlorid klar löslich ist, mit einem durch Gelpermeationschromatographie (in Tetrahydrofuran) ermittelten Molekulargewicht von 8660 (Mn) und 55570 (Mw).
b) In eine Suspension von 4 g 10%-iger Pd-Kohle in 500 ml Dimethylformamid werden 100 g der unter a) beschriebenen Nitroverbindung während 20 Stunden bei 65°C in Gegenwart von Wasserstoff (Normaldruck) hydriert. Anschliessend wird die Reaktionslösung filtriert und das Filtrat in 5 Liter Wasser ausgefällt. Der Niederschlag wird abfiltriert, ein zweites Mal mit 5 Liter Wasser gemischt, filtriert und im Vakuum bei 80°C getrocknet. Man erhält 76 g eines violetten Pulvers mit einem Amingehalt von 2,7 Aequivalenten/kg, das in Methylenchlorid gut löslich ist (Mn: 8600, Mw: 55700).

Beispiel 2:

a) Analog Beispiel 1 werden 144 g (0,75 Mol) 1,3-Dichlor-4-nitrobenzol, 110,1 g (1 Mol) Resorcin, 290 g (2,1 Mol) wasserfreies, pulverisiertes Kaliumcarbonat, 350 ml NMP und 700 ml Toluol umgesetzt. Man erhält 185 g eines braunen Pulvers, das in Methylenchlorid klar löslich ist, mit einem durch Gelpermeationschromatographie (in Tetrahydrofuran) ermittelten Molekulargewicht von 670 (Mn) und 1110 (Mw).
b) 160 g der unter a) erhaltenen Nitroverbindung werden, wie in Beispiel 1b) beschrieben, mit Wasserstoff zum Amin reduziert. Man erhält 95 g eines dunkelbraunen Pulvers mit einem Amingehalt von 3,3 Aequivalenten/kg, einem Mn von 646 und Mw von 1052 und einem Phenolgehalt von 2,8 Aequivalenten/kg.

Beispiel 3:

a) 221 g (0,77 Mol) Dichlordiphenylsulfon, 191,5 g (0,84 Mol) Bisphenol A, 9,6 g (0,05 Mol) 1,3-Dichlor-4-nitrobenzol, 261 g (1,9 Mol) wasserfreies Kaliumcarbonat, 800 ml Dimethylacetamid und 400 ml Xylol werden gemäss Beispiel 1a) zur Reaktion gebracht und analog aufgearbeitet.
Man erhält 309 g eines gelben Pulvers, das in Methylenchlorid gut löslich ist.
Gelpermeationschromatographie: Mn = 8050;MW = 28400.
b) In eine Suspension von 30 g 10%iger Pd-Kohle in 2500 ml Dimethylformamid werden 300 g der unter a) beschriebenen Nitroverbindung während 3 Stunden bei 35°C in Gegenwart von Wasserstoff (Normaldruck) hydriert. Anschliessend wird die Reaktionslösung filtriert und das Filtrat in 15 Liter Wasser ausgefällt. Der Niederschlag wird abfiltriert, ein zweites Mal mit 10 Liter Wasser gemischt, filtriert und im Vakuum bei 80°C getrocknet. Man erhält 242 g eines violetten Pulvers mit einem Amingehalt von 0,13 Aequivalenten/kg, das in Methylenchlorid gut löslich ist und eine $\eta_{inh}$ von 0,28 aufweist.
Gelpermeationschromatographie: Mn = 8100; MW = 27800.

Beispiel 4:

a) 222,4 g (0,775 Mol) Dichlordiphenylsulfon, 239,4 g (1,05 Mol) Bisphenol A, 48,0 g (0,25 Mol) 1,3-Dichlor-4-nitrobenzol, 325,8 g (2,36 Mol) wasserfreies Kaliumcarbonat, 1 Liter Dimethylacetamid und 400 ml Xylol werden gemäss Beispiel 1a) zur Reaktion gebracht und analog aufgearbeitet.
Man erhält 408 g eines gelben Pulvers, das in Methylenchlorid gut löslich ist und eine $\eta_{inh}$ von 0,20 aufweist.
Gelpermeationschromatographie: Mn = 6670; MW = 17260.
b) Wie in Beisiel 3b) beschrieben werden 380 g der unter 4a) erhaltenen Nitroverbindung mit Wasserstoff zum Amin reduziert. Man erhält 167 g eines violetten Pulvers mit einem Amingehalt von 0,41 Aequivalenten/kg und einer $\eta_{inh}$ von 0,25.
Gelpermeationschromatographie: Mn = 6500; MW = 17800.

Beispiel 5:

In einem 750 ml Sulfierkolben werden 57,2 g (0,20 Mol) Dichlordiphenyl-sulfon, 39,9 g (0,175 Mol) Bisphenol A, 7,96 g (0,025 Mol) 2,4-Bis-(4-hydroxyphenoxy)-anilin, 33,1 g (0,24 Mol) wasserfreies Kaliumcarbonat, 400 ml Dimethylacetamid und 200 ml Xylol bei 146°C zur Reaktion gebracht und das entstehende Reaktionswasser wird laufend azeotrop ausgekreist. Nach 12 Stunden wird das Xylol aus dem Reaktionsgefäss abdestilliert und die verbleibende Suspension nach Abkühlen auf Raumtemperatur auf 3 Liter Wasser gegossen. Der Niederschlag wird abfiltriert, ein zweites Mal mit 3 Liter Wasser gemischt, filtriert, mit Wasser gut nachgewaschen und im Vakuum bei 100°C getrocknet. Man erhält 74,8 g eines violetten Pulvers mit einem Amingehalt von 0,27 Aequivalenten/kg, das in Methylenchlorid gut löslich ist und eine $\eta_{inh}$ von 0,64 aufweist.
Gelpermeationschromatographie: Mn = 11500; MW = 17300.

Beispiel 6:

a) In einem 2,5 Liter Sulfierkolben werden 196,1 g (0,86 Mol) Bisphenol A, 3 8,4 g (0,20 Mol) 1,3-Dichlor-4-nitrobenzol, 255,4 g (1,84 Mol) wasserfreies Kaliumcarbonat, 1,2 Liter Dimethylacetamid und 400 ml Toluol bei 140°C zur Reaktion gebracht und das entstehende Reaktionswasser wird laufend azeotrop ausgekreist. Nach 4 Stunden wird auf 100°C abgekühlt und 139,5 g (0,64 Mol) Difluorbenzophenon werden zugegeben. Anschliessend erhöht man die Innentemperatur wieder auf 140°C und lässt 12 Stunden weiterreagieren. Das Xylol wird dann aus dem Reaktionsgefäss abdestilliert und die verbleibende Suspension wird nach Abkühlen auf Raumtemperatur auf 10 Liter Wasser gegossen. Der Niederschlag wird abfiltriert, ein zweites Mal mit 10 Liter Wasser gemischt, filtriert, mit Wasser gut nachgewaschen und im Vakuum bei 100°C getrocknet. Man erhält 295 g eines hellgelben Pulvers, das in Methylenchlorid Klar löslich ist, mit einem durch Gelpermeationschromatographie (in Tetrahydrofuran) ermittelten Molekulargewicht von 9000 (Mn) und 45550 (Mw). $\eta_{inh}$ = 0,34.
b) In eine Suspension von 86 g 5%iger Pd-Cacliumcarbonat in 5 Liter Dimethylformamid werden 280 g der unter a) beschriebenen Nitroverbindung während 10 Stunden bei Raumtemperatur in Gegenwart von Wasserstoff (Normaldruck) hydriert. Anschliessend wird die Reaktionslösung filtriert und das Filtrat in 20 Liter Wasser ausgefällt. Der Niederschlag wird abfiltriert, ein zweites Mal mit 5 Liter Wasser gemischt, filtriert und im Vakuum bei 80°C getrocknet. Man erhält 260 g eines violetten Pulvers mit einem Amingehalt von 0,43 Aeq./kg, das in Methylenchlorid gut löslich ist. $\eta_{inh}$ = 0,37.
Gelpermeationschromatographie: Mn = 8600; MW = 38500.

Anwendungsbeispiele

Beispiel I:

Zu einer Epoxidharz/Härter-Kombination bestehend aus 50 g N,N,N',N'-Tetra-glycidyl-p-diaminodiphenylmethan, 50 g N,N,O-Triglycidyl-p-aminophenol und 50 g p-Diaminodiphenylsulfon gelöst in 100 ml Methylenchlorid werden 10 g oder 20 g Polymer gemäss Beispiel 3b) eingebracht. Das Lösungsmittel wird dann bei 150°C am Rotationsverdampfer abdestilliert. Die bei 150°C gut giessbare Harzmischung wird in eine 4 mm dicke Metallform gegossen und 2 Stunden bei 160°C und 2 Stunden bei 210°C gehärtet. Nach dem Entformen erhält man eine rotbraune, transparente Platte mit folgenden Eigenschaften:

| | Polymer gemäss Beispiel 3b) 10 g | 20 g |
|---|---|---|
| Tg (TMA) | 246°C | 244°C |
| Biegefestigkeit (nach ISO 178) | 138 MPa | 150 MPa |
| Randfaserdehung (nach ISO 178) | 4,2 % | 5,0 % |
| Bruchzähigkeit (nach ASTM E 399-789) | 100 J/m² | 164 J/m² |

Beispiel II:

Zu einer Epoxidharz/Härter-Kombination bestehend aus 50 g N,N,N',N'-Tetra-glycidyl-p-diaminodiphenylmethan, 50 g N,N,O-Triglycidyl-p-aminophenol und 50 g p-Diaminodiphenylsulfon gelöst in 100 ml Methylenchlorid werden 10 g oder 30 g Polymer gemäss Beispiel 4b) eingebracht. Das Lösungsmittel wird dann bei 150°C am Rotationsverdampfer abdestilliert. Die bei 150°C gut giessbare Harzmischung wird in eine 4 mm dicke Metallform gegossen und 2 Stunden bei 160°C und 2 Stunden bei 210°C gehärtet. Nach dem Entformen erhält man eine rotbraune, transparente Platte an der folgende Polymereigenschaften gemessen werden:

| | Polymer gemäss Beispiel 4b) 10 g | 30 g |
|---|---|---|
| Tg (TMA) | 246°C | 239°C |
| Biegefestigkeit (nach ISO 178) | 156 MPa | 146 MPa |
| Randfaserdehung (nach ISO 178) | 5,5 % | 5,1 % |
| Bruchzähigkeit (nach ASTM E 399-789) | 164 J/m² | 219 J/m² |

Beispiel III:

Zu einer Epoxidharz/Härter-Kombination bestehend aus 100 g N,N,N',N'-Tetraglycidyl-4,4'-diamino-3,3'-diäthyldiphenylmethan und 45 g p-Diaminodiphenylsulfon, gelöst in 100 ml Methylenchlorid, werden 10 g oder 30 g Polymer gemäss Beispiel 4b) eingebracht. Das Lösungsmittel wird dann bei 150°C am Rotationsverdampfer abdstilliert. Die bei 150°C gut giessbare Harzmischung wird in eine 4 mm dicke Metallform gegossen und 4 Stunden bei 180°C gehärtet. Nach dem Entformen erhält man eine rotbraune, transparente Platte, an der folgende Polymereigenschaften gemessen werden:

| | Polymer gemäss Beispiel 4b) 10 g | 30 g |
|---|---|---|
| Tg (TMA) | 212°C | 192°C |
| Biegefestigkeit (nach ISO 178) | 112 MPa | 120 MPa |
| Randfaserdehung (nach ISO 178) | 3,3 % | 3,9 % |

Beispiel IV:

Zu einer Epoxidharz/Härter-Kombination bestehend aus 40 g N,N,N',N'-Tetraglycidyl-4,4'-diamino-diphenylmethan und 40 g N,N,O-Triglycidyl-p-aminophenol und 36 g p-Diaminodiphenylmethan, gelöst in 100 ml Methylenchlorid, werden 8 g Polymer gemäss Beispiel 6b) eingebracht. Das Lösungsmittel wird dann bei 120°C

am Rotationsverdampfer abdstilliert. Die bei 150°C gut giessbare Harzmischung wird in eine 4 mm dicke Metallform gegossen und 2 Stunden bei 160°C und 2 Stunden bei 210°C gehärtet. Nach dem Entformen erhält man eine rotbraune, transparente Platte an der folgende Polymereigenschaften gemessen werden:

| | Polymer gemäss Beispiel 6b) 8 g |
|---|---|
| Tg (TMA) | 230°C |
| Biegefestigkeit (nach ISO 178) | 115 MPa |
| Randfaserdehung (nach ISO 178) | 3,9 % |
| Bruchzähigkeit (nach ASTM E 399-789) | 176 J/m² |

## Patentansprüche

1. In organischen Lösungsmitteln lösliche, Aminogruppen aufweisende Polyarylenether mit einer inhärenten Viskosität ($\eta_{inh}$) von 0,02 bis 1,0, gemessen an einer 1 gew.-%igen Lösung in N-Methylpyrrolidon bei 25°C, die, bezogen auf die Gesamtmenge der im Polymeren vorhandenen Strukturelemente, 100 bis 5 Mol-% eines wiederkehrenden Strukturelementes der Formel I oder II

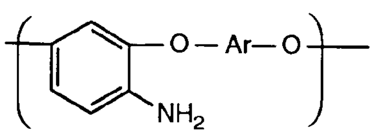

(I)

oder

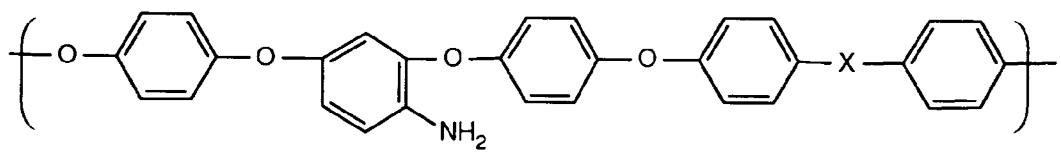

(II)

und 95 bis 0 Mol-% eines wiederkehrenden Strukturelementes der Formel III

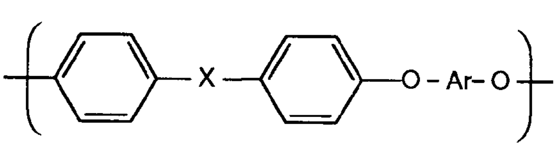

(III)

enthalten, worin
X für - $SO_2$- oder -CO- steht und
Ar eine unsubstituierte oder durch ein oder mehrere $C_1$ -$C_4$-Alkyle, $C_1$-$C_4$ -Alkoxy oder Halogenatome substituierte Gruppe der Formeln IVa bis IVe

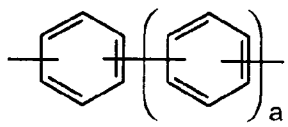
(IVa),

wobei a Null oder die Zahl 1 ist,

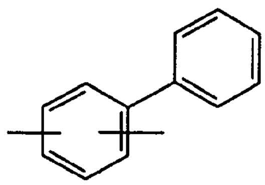
(IVb),
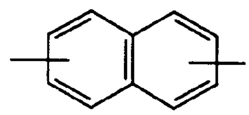
(IVc),

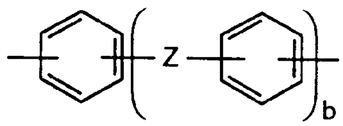
(IVd),

wobei b die Zahl 1 oder 2 ist, oder

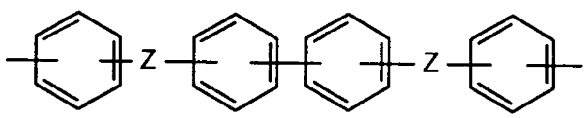
(IVe)

darstellt, worin Z für

$$-CO-,\ -SO_2-,\ -SO-,\ -S-,\ -O-,\ -\overset{|}{C}(CH_3)_2\ ,\ -\overset{|}{C}(CF_3)_2\ ,\ -CH_2-$$

oder

$$-\overset{|}{\underset{\underset{C_6H_5}{|}}{C}}-CH_3$$ steht.

2. Polyarylenether gemäss Anspruch 1, enthaltend 100 bis 5 Mol-% eines wiederkehrenden Strukturelementes der Formel I und 95 bis 0 Mol-% eines wiederkehrenden Strukturelementes der Formel III.

3. Polyarylenether gemäss Anspruch 1, worin Ar in den Formeln I und III eine unsubstituierte Gruppe der Formeln IVa bis IVe bedeutet.

4. Polyarylenether gemäss Anspruch 1, worin Ar in den Formeln I und III für einen Rest der Formeln

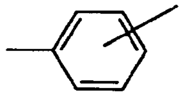 , 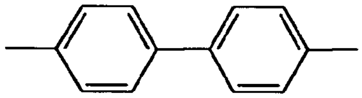 , 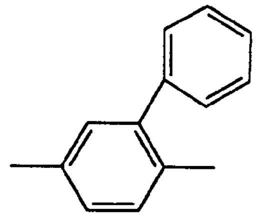 ,

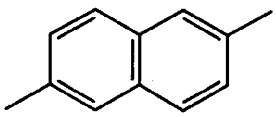 , 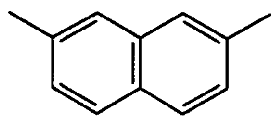 , 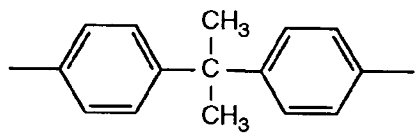

oder

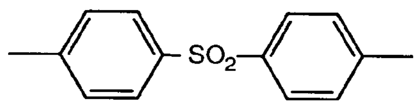

steht.

5. Polyarylenether gemäss Anspruch 1, worin Ar in den Formeln I und III für einen Rest der Formeln

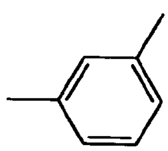 , 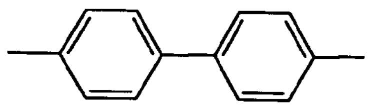 , 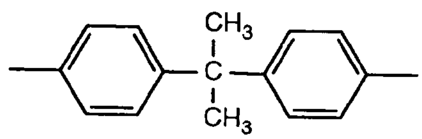

oder

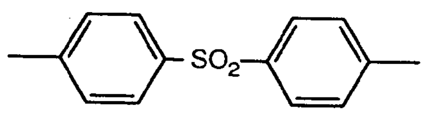

steht.

6. Verfahren zur Herstellung von Polyarylenether gemäss Anspruch 1, dadurch gekennzeichnet, dass man 1,3-Dichlor-4-nitrobenzol oder ein Gemisch aus 1,3-Dichlor-4-nitrobenzol und einer darin bis zu 95 Mol-% enthaltenen Dihalogenverbindung der Formel V

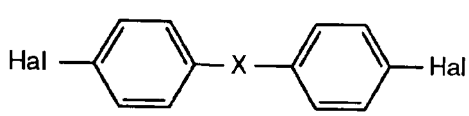 (V),

worin Hal für ein Halogenatom, vorzugsweise Chlor oder Fluor, steht und X die gleiche Bedeutung wie in Anspruch 1 hat, mit einem Diphenol der Formel VI

HO-Ar-OH (VI)

worin Ar die gleiche Bedeutung wie in Anspruch 1 hat, oder dass man 2,4-Bis-4-(hydroxyphenoxy)-anilin oder ein Gemisch aus 2,4-Bis-(4-hydroxyphenoxy)-anilin und einem darin bis zu 95 Mol-% enthaltenen Diphenol der Formel VI mit einer Halogenverbindung der Formel V in Gegenwart von Alkali und in einem aprotischen Lösungsmittel polykondensiert, bis der erhaltene Polyarylenether eine $\eta_{inh}$ von 0,02 bis 1,0

aufweist, und anschliessend den Nitrogruppen enthaltenden Polyarylenether in an sich bekannter Weise durch katalytische Reduktion der Nitrogruppen in einen Aminogruppen aufweisenden Polyarylenether gemäss Anspruch 1 umwandelt.

**7.** 2,4-Bis-(4-hydroxyphenoxy)-anilin.

**8.** Verwendung der Polyarylenether gemäss Anspruch 1 zum Härten oder Modifizieren von Epoxidharzen oder Maleinimidharzen.

**9.** Härtbares Gemisch, enthaltend
(a) ein Epoxidharz mit mehr als einer Epoxidgruppe im Molekül und
(b) einen Polyarylenether gemäss Anspruch 1 in für die Aushärtung des Epoxidharzes ausreichender Menge.

**10.** Härtbares Gemisch gemäss Anspruch 9, enthaltend ausserdem ein von (b) verschiedenes Epoxidharzhärtungsmittel (c), wobei (b) und (c) zusammen in für die Aushärtung des Epoxidharzes ausreichender Menge vorliegen.

**11.** Härtbares Gemisch gemäss Anspruch 10, enthaltend die komponenten (a), (b) und (c), wobei das Härtungsmittel (c) in für die Aushärtung des Epoxidharzes ausreichender Menge vorliegt und der Polyarylenether (b) in Mengen bis zu 50 Gew.%, bezogen auf die Menge (a) und (c), vorliegt.

**Patentansprüche für folgenden Vertragsstaat: ES**

**1.** Verfahren zur Herstellung von in organischen Lösungsmitteln löslichen, Aminogruppen aufweisenden Polyarylenethern mit einer inhärenten Viskosität ($\eta_{inh}$) von 0,02 bis 1,0, gemessen an einer 1 gew.-%igen Lösung in N-Methylpyrrolidon bei 25°C, die, bezogen auf die Gesamtmenge der im Polymeren vorhandenen Strukturelemente, 100 bis 5 Mol-% eines wiederkehrenden Strukturelementes der Formel I oder II

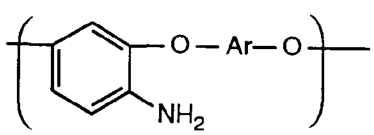

(I)

oder

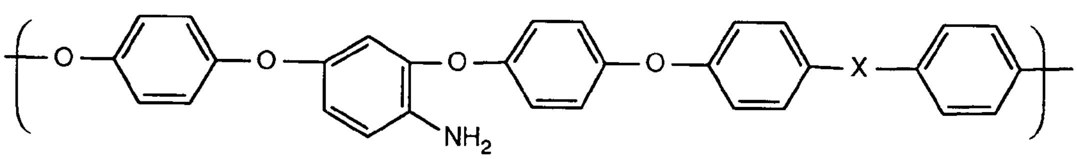

(II)

und 95 bis 0 Mol-% eines wiederkehrenden Strukturelementes der Formel III

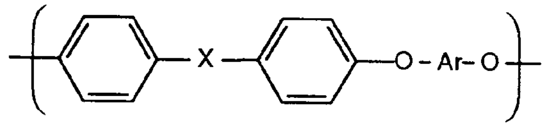

(III)

enthalten, worin X für $-SO_2-$ oder -CO- steht und
Ar eine unsubstituierte oder durch ein oder mehrere $C_1$-$C_4$-Alkyle, $C_1$-$C_4$-Alkoxy oder Halogenatome substituierte Gruppe der Formeln IVa bis IVe

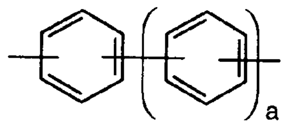 (IVa),

wobei a Null oder die Zahl 1 ist,

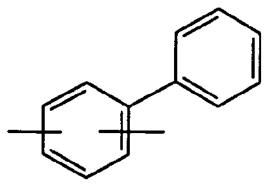 (IVb), 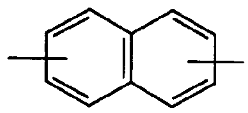 (IVc),

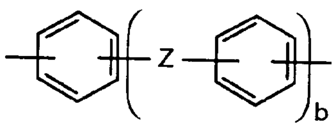 (IVd),

wobei b die Zahl 1 oder 2 ist, oder

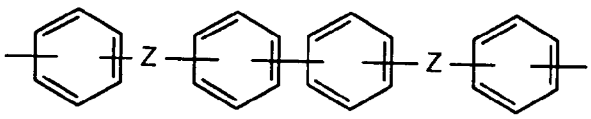 (IVe)

darstellt, worin Z für

-CO-, $-SO_2-$, -SO-, -S-, -O-, $-C(CH_3)_2$ , $-C(CF_3)_2$ , $-CH_2-$

oder

$-C(CH_3)(C_6H_5)-$

steht, dadurch gekennzeichnet, dass man 1,3-Dichlor-4-nitrobenzol oder ein Gemisch aus 1,3-Dichlor-4-nitrobenzol und einer darin bis zu 95 Mol-% enthaltenen Dihalogenverbindung der Formel V

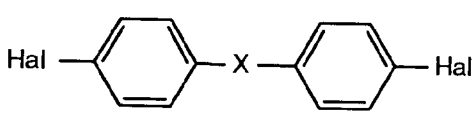 (V),

worin Hal für ein Halogenatom steht und X die gleiche Bedeutung wie in Formel II oder III hat, mit einem Diphenol der Formel VI

HO-Ar-OH (VI),

worin Ar die gleiche Bedeutung wie in Formel I hat, oder dass man 2,4-Bis-4-(hydroxyphenoxy)-anilin oder ein Gemisch aus 2,4-Bis-(4-hydroxyphenoxy)-anilin und einem darin bis zu 95 Mol-% enthaltenen Diphenol der Formel VI mit einer Halogenverbindung der Formel V in Gegenwart von Alkali und in einem aprotischen Lösungsmittel polykondensiert, bis der erhaltene Polyarylenether eine $\eta_{inh}$ von 0,02 bis 1,0 aufweist, und anschliessend den Nitrogruppen enthaltenden Polyarylenether in an sich bekannter Weise durch katalytische Reduktion der Nitrogruppen in einen Aminogruppen aufweisenden Polyarylenether mit den wiederkehrenden Strukturelementen der Formel I oder II und III umwandelt.

**2.** Härtbares Gemisch, enthaltend
(a) ein Epoxidharz mit mehr als einer Epoxidgruppe im Molekül und
(b) einen gemäss Anspruch 1 hergestellten Polyarylenether in für die Aushärtung des Epoxidharzes ausreichender Menge.

**3.** Härtbares Gemisch gemäss Anspruch 2, enthaltend ausserdem ein von (b) verschiedenes Epoxidharzhärtungsmittel (c), wobei (b) und (c) zusammen in für die Aushärtung des Epoxidharzes ausreichender Menge vorliegen.

**4.** Härtbares Gemisch gemäss Anspruch 3, enthaltend die komponenten (a), (b) und (c), wobei das Härtungsmittel (c) in für die Aushärtung des Epoxidharzes ausreichender Menge vorliegt und der Polyarylenether (b) in Mengen bis zu 50 Gew.%, bezogen auf die Menge (a) und (c), vorliegt.